# EUROPÄISCHE PATENTSCHRIFT

(11) **EP 0 626 935 B1**
(45) Veröffentlichungstag und Bekanntmachung des Hinweises auf die Patenterteilung: **11.10.1995**
(21) Anmeldenummer: 93909969.3
(22) Anmeldetag: 12.05.1993
(51) Int. Cl.: C07C 7/00

(54) **VERFAHREN ZUM AUFTRENNEN EINES IM WESENTLICHEN AUS WASSERSTOFF, METHAN UND C 3/C 4-KOHLENWASSERSTOFFEN BESTEHENDEN EINSATZSTROMES.**
PROCESS FOR SEPARATING AN INTAKE FLOW CONSISTING ESSENTIALLY OF HYDROGEN, METHANE AND C 3/C 4 HYDROCARBONS
PROCEDE DE SEPARATION D'UN COURANT DE CHARGE CONSTITUE ESSENTIELLEMENT D'HYDROGENE, DE METHANE ET D'HYDROCARBURES C 3/C 4

(30) Priorität: 16.10.1992 DE 4235006
(43) Veröffentlichungstag der Anmeldung: 07.12.1994
(73) Patentinhaber: Linde Aktiengesellschaft, 65189 Wiesbaden (DE)
(72) Erfinder: BAUER, Heinz, D-8000 München 71 (DE)
(74) Vertreter: Kasseckert, Rainer
(86) Internationale Anmeldenummer: EP9301182
(87) Internationale Veröffentlichungsnummer: WO9408923

(56) Entgegenhaltungen:
- EP-A- 0 153 984
- EP-A- 0 182 643
- WO-A-88/00936

## Beschreibung

Die Erfindung betrifft ein Verfahren zum Auftrennen eines im wesentlichen aus Wasserstoff, Methan und C₃/C₄-Kohlenwasserstoffen bestehenden Einsatzstromes, der zuvor eine Dehydrierung durchgeläufen ist (Vorbehandlung), nachdem der Einsatzstrom eine mindestens einstufige partielle Kondensation und Auftrennung in eine C₃/C₄-Kohlenwasserstoff-reiche Fraktion und in eine H₂/CH₄-Fraktion erfährt, wonach ein Teilstrom der H₂/CH₄-Fraktion dem noch nicht vorbehandelten Einsatzstrom beigemischt wird.

Verfahren zum Auftrennen eines im wesentlichen aus Wasserstoff, Methan und C₃/C₄-Kohlenwasserstoffen bestehenden Einsatzstromes finden z.B. bei der einer Dehydrierung von Iso-Butan zu Iso-Buten nachgeschalteter kryogenen Zerlegung des aus dem Dehydrierungsreaktor stammenden Produktstromes Verwendung. Die Dehydrierung von Iso-Butan zu Iso-Buten spielt vor allem bei der Herstellung von MTBE (Methyl-tert.-Butylether) eine wichtige Rolle, da zur Herstellung von MTBE Iso-Buten und Methanol gebraucht werden. Aufgrund von sich zunehmend verschärfenden Abgasvorschriften für Kraftfahrzeuge wird der Verbrauch an MTBE, das als Oktanzahl-Verbesserer für Kraftstoffe verwendet wird, in den folgenden Jahren ansteigen. Während Methanol in ausreichenden Mengen zur Verfügung steht, besteht jedoch an Iso-Buten ein erheblicher Mangel.

Einen Überblick über die zum Stand der Technik zählenden Verfahrensmöglichkeiten zur Gewinnung von C₃/C₄-Kohlenwasserstoff-reichen Produktfraktionen gibt der Artikel "Cryogenic Olefins Recovery from Dehydrogenation Reactor Effluents" von Dr. Heinz C. Bauer (präsentiert auf dem AIChE-Symposium on Cryogenic Gas Processing, 1992 Spring National Meeting, 29.03-02.04.92). Speziell Figur 13 zeigt hierbei ein Verfahren zum Zerlegen eines im wesentlichen aus Wasserstoff, Methan und C₄-Kohlenwasserstoffen bestehenden Einsatzstromes, bei dem ein Teil des gewonnenen Gasgemisches, bestehend aus Wasserstoff und Methan, aus der Anlage abgeführt (Net fuel gas) und ein Teil in den Dehydrierreaktor (Recyclegas) zurückgeführt wird. Diese Rückführung eines Teiles des Gasgemisches zum Dehydrierungsreaktor geschieht, um in ihm eine unerwünschte Rußbildung zu vermindern. Dies gelingt jedoch nur unvollständig.

Erfindungsgemäß wurde nunmehr festgestellt, daß sich die unerwünschte Rußbildung umso besser unterdrücken läßt, je höher der Wasserstoffanteil im zu dem Dehydrierungsreaktor zurückgeführten Strom ist. Zusätzliche Stoffe, wie z.B Methan, stellen, wie neueste, der Erfindung zugrundeliegende Untersuchungen ergeben haben, nur einen unnötigen Ballast dar, der sowohl die Kapazität blockiert als auch die Wirtschaftlichkeit des Verfahrens verschlechtert.

Ziel und Aufgabe der vorliegenden Erfindung ist es, ein Verfahren anzugeben, bei dem unter anderem ein H₂-reicher Gasstrom in die Dehydrierung zurückgeführt und ein, die für das Verfahren als inerten Ballaststoff zu betrachtenden Komponenten enthaltender Gasstrom gewonnen wird.

Dies wird erfindungsgemäß dadurch erreicht, daß die aus dem ersten Abscheider abgezogene H₂/CH₄-Gasfraktion in zwei Teilströme aufgeteilt wird, wobei der erste Teilstrom zusammen mit der Flüssigfraktion des ersten Abscheiders nach partieller Kondensation einem zweiten Abscheider zugeführt, die am Sumpf des zweiten Abscheiders abgezogene C₃/C₄-Kohlenwasserstoff-reiche Flüssigfraktion zusammen mit dem zweiten Teilstrom der H₂/CH₄-Gasfraktion aus dem ersten Abscheider in eine Stripperkolonne geleitet wird, daß am Kopf des zweiten Abscheiders eine H₂-reiche Gasfraktion mit hoher Reinheit abgezogen und nach Erwärmen im Wärmetausch gegen abzukühlende Verfahrensströme mit dem Einsatzstrom vor dessen Vorbehandlung vermischt wird und daß am Sumpf der Stripperkolonne eine C₃/C₄-Kohlenwasserstoff-reiche Produktfraktion und am Kopf der Stripperkolonne eine H₂-reiche Gasfraktion mit weniger hoher Reinheit abgezogen werden.

Mittels der Zugabe der flüssigen Sumpffraktion des ersten Abscheiders zu dem ersten Teilstrom der aus dem ersten Abscheider abgezogenen H₂/CH₄-Gasfraktion wird bereits in dem zweiten Abscheider die gewünschte Wasserstoffqualität in der Gasfraktion erreicht. Während nämlich bei den zum Stand der Technik zählenden Verfahren nur die flüssigen C₃/C₄-reichen Sumpffraktionen der einzelnen Abscheider zusammengeführt werden, wird bei dem erfindungsgemäßen Verfahren die flüssige C₃/C₄-reiche Sumpffraktion des ersten Abscheiders als Lösungsmittel für die noch im ersten Teilstrom enthaltenen C₃/C₄-Kohlenwasserstoffe verwendet. Auf diese Weise kann die für das Kreislaufgas benötigte Wasserstoffreinheit bereits bei höheren Temperaturen erzielt werden. Darüber hinaus wird bei dem erfindungsgemäßen Verfahren nur der Teilstrom der Kopffraktion des ersten Abscheiders von inerten Komponenten befreit, der letztendlich dem Dehydrierungsreaktor wieder zugeführt wird.

Die am Sumpf des zweiten Abscheiders abgezogene Flüssigfraktion wird nach Erwärmung und gegebenenfalls teilweiser Verdampfung zusammen mit dem zweiten Teilstrom des aus dem ersten Abscheider abgezogenen H₂/CH₄-Gasstromes in eine Stripperkolonne geleitet. In dieser erfolgt dann die Auftrennung in eine C₃/C₄-reiche Produktfraktion und eine weitere, hauptsächlich mit Methan verunreinigte H₂-reiche Gasfraktion.

Das am Kopf des zweiten Abscheiders abgezogene H₂-reiche Kreislaufgas mit hoher Reinheit wird zunächst gegebenenfalls kälteleistend entspannt, um die für den Prozess benötigte Spitzenkälte bereitstellen zu können. Die bei dieser Entspannung anfallende mechanische Energie kann z.B. zum Vorverdichten des Einsatzstromes verwendet werden. Nach dem Anwärmen im Wärmetausch gegen abzukühlende Verfahrensströme wird das H₂-reiche Kreislaufgas mit dem noch nicht vorbehandelten Einsatzstrom vermischt und vor die Dehydrierung zurückgeführt.

Die Erfindung weiterbildend wird vorgeschlagen, daß das Vermischen der am Kopf des zweiten Abscheiders abgezogenen H₂-reichen Gasfraktion mit hoher Reinheit mit dem noch nicht vorbehandelten Einsatzstrom auf mindestens zwei unterschiedlichen Temperaturniveaus erfolgt.

Die Kälteversorgung des Gesamtprozesses erfolgt auf energetisch sinnvolle Weise mittels Verdampfen des flüssigen noch nicht vorbehandelten Einsatzstromes. Dabei wird die Verdampfung in der Regel in mehreren Stufen durchgeführt. Die Vermischung des noch nicht vorbehandelten Einsatzstromes mit dem entspannten H₂-reichen Gasstrom aus dem zweiten Abscheider erfolgt nun nicht auf einmal, z.B. auf dem tiefsten Temperaturniveau, sondern in mehreren Stufen. Dadurch wird die bei gleitender Temperatur stattfindende Kondensation des Einsatzstromes besser unterstützt.

Eine weitere Ausgestaltung des erfindungsgemäßen Verfahrens ist dadurch gekennzeichnet, daß unterhalb des Kopfes der Stripperkolonne eine H₂-reiche Gasfraktion abgezogen, teilweise kondensiert und oberhalb der Abzugstelle wieder auf den Kopf der Kolonne gegeben wird.

Mittels dieser Ausgestaltung des erfindungsgemäßen Verfahrens wird eine Reduzierung der C₃/C₄-Produktverluste am Sumpf der Stripperkolonne erzielt.

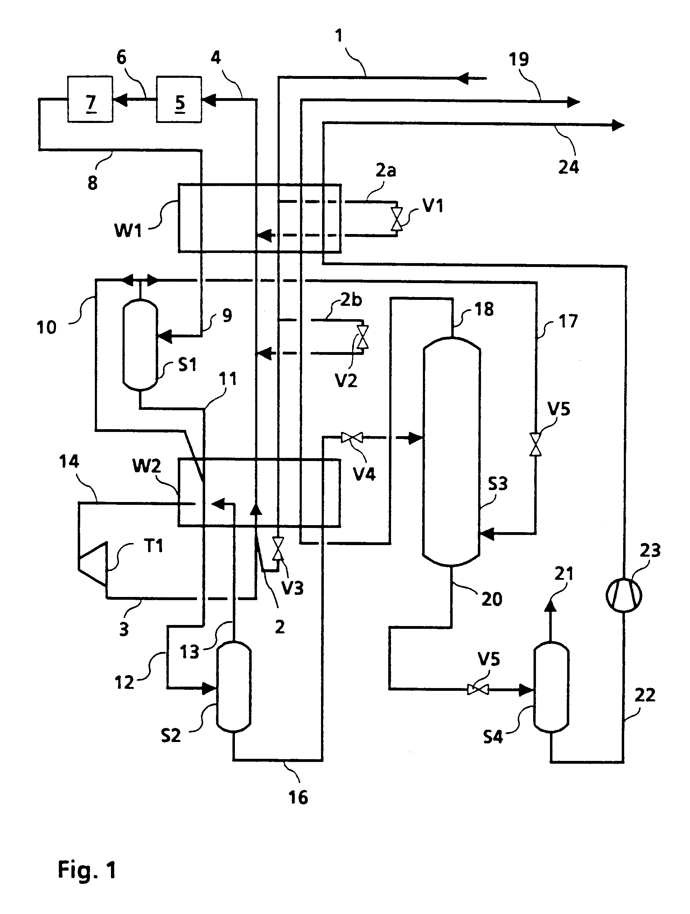

Die Erfindung sowie weitere Ausgestaltungen der Erfindung sind anhand des Ausführungsbeispieles der Figur 1 dargestellt.

Über Leitung 1 wird ein Gasgemisch bestehend aus 95,2 % i-C₄H₁₀, 2,9 % C₄H₁₀ und 1,9 % sonstigen Komponenten (alle Angaben beziehen sich auf Mol-%) mit einer Temperatur von 43,0°C und einem Druck von 10,7 bar in den Prozess geführt. Der Einsatzstrom wird nun in den beiden Wärmetauschern W1 und W2 zur Bereitstellung der Prozesskälte verdampft und anschließend dem entspannten H₂-reichen Gas in Leitung 3 (auf das später noch näher eingegangen wird) beigemischt. Diese Beimischung erfolgt sinnvollerweise auf drei unterschiedlichen Temperaturniveaus, nämlich im Wärmetauscher W1 (über Leitung 2a), zwischen den beiden Wärmetauschern W1 und W2 (über Leitung 2b) und nach bzw. im Wärmetauscher W2 (über Leitung 2). Das Abgleichen der drei Zumischmöglichkeiten geschieht mittels der Ventile V1, V2 und V3. Der nun im wesentlichen Wasserstoff, Methan und C₄-Kohlenwasserstoffe enthaltende Einsatzstrom (44,6 % H₂, 5,8 % CH₄, 47,2 % i-C₄H₁₀ und 2,4 % sonstige Komponenten) wird in den Wärmetauschern W2 und W1 erwärmt und über Leitung 4 mit einer Temperatur von 50,7°C und einem Druck von 2,5 bar in den Dehydrierreaktor 5 geleitet. Der aus dem Dehydrierreaktor 5 austretende Einsatzstrom wird zunächst über Leitung 6 einer Verdichter- und Trocknungseinheit 7 zugeführt und anschließend über Leitung 8 mit einer Zusammensetzung von 51,7 % H₂, 9,0 % CH₄, 19,3 % i-C₄H₁₀, 15,8 % i-C₄H₈ und 4,2 % sonstigen Komponenten und mit einer Temperatur von 54°C und einem Druck von 6,6 bar in den Wärmetauscher W1 geführt. In ihm wird dieser Strom teilweise kondensiert und anschließend über Leitung 9 Abscheider S1 zugeleitet. Am Kopf des Abscheiders S1 wird eine gasförmige Fraktion mit einer Zusammensetzung von 84,8 % H₂, 13,8 % CH₄ und 2,4 % sonstige Komponenten und mit einer Temperatur von -67°C und einem Druck von 6,5 bar abgezogen und über Leitung 10 der Flüssigfraktion aus dem Abscheider S1 zugemischt. Diese Flüssigfraktion besteht aus 48,7 % i-C₄H₁₀, 39,8 % i-C₄H₈ und 11,5 % sonstigen Komponenten. Nach einer Abkühlung im Wärmetauscher W2 auf -116,3°C wird dieser teilkondensierte Strom über Leitung 12 mit einem Druck von 6,3 bar in den Abscheider S2 geleitet. In ihm erfolgt eine Auftrennung in eine Gasfraktion, bestehend aus 88,5 % H₂ und 11,5 % CH₄, sowie in eine flüssige Fraktion, bestehend aus 47,0 % i-C₄H₁₀, 4,5 % CH₄, 38,4 % i- C₄H₈ und 10,1 % sonstigen Komponenten. Am Kopf des Abscheiders S2 wird die Gasfraktion über Leitung 13 abgezogen, im Wärmetauscher W2 angewärmt und über Leitung 14 der Entspannungsturbine T1 zugeführt. Der Einfachheit halber ist in der Figur nur eine Entspannungsturbine dargestellt; es ist jedoch für den Fachmann selbstverständlich, daß auch eine mehrstufige Entspannung stattfinden kann. Die H₂-reiche Gasfraktion wird in der Entspannungsturbine T1 auf einen Druck von 2,5 bar entspannt und anschließend über Leitung 3 der ersten der drei Zumischstellen des noch nicht vorbehandelten Einsatzstromes zugeführt. Die am Sumpf des Abscheiders S2 abgezogene C₄-Kohlenwasserstoff-reiche Flüssigfraktion wird über Leitung 16 in den Wärmetauscher W2 geführt, in ihm erwärmt und anschließend über Ventil V4 in den mittleren Bereich der Stripperkolonne S3 entspannt. Zusätzlich zu dieser Fraktion wird über Leitung 17 und Ventil V5 der zweite Teil der am Kopf des ersten Abscheiders S1 abgezogenen H₂-reichen Gasfraktion im Bereich des Bodens der Stripperkolonne S3 aufgegeben. Am Kopf der Stripperkolonne S3 fällt eine H₂-reiche, CH₄-enthaltende Gasfraktion an. Diese wird über Leitung 18 den Wärmetauschern W2 und W1 zugeführt, in ihnen im Wärmetausch gegen abzukühlende Verfahrensströme erwärmt und anschließend mit einer Zusammensetzung von 80,0 % H₂, 19,3 % CH₄ und 0,7 % sonstigen Komponenten und mit einer Temperatur von 50,7°C und einem Druck von 5,6 bar über Leitung 19 aus der Anlage abgeführt. Die am Sumpf der Strippersäule S3 anfallende C₄-reiche Fraktion wird über Leitung 20 und Ventil V5 in den Abscheider S4 entspannt. Am Kopf des Abscheiders S4 wird über Leitung 21 eine Gasfraktion mit einer Zusammensetzung von 32,9% H₂, 59,7 % CH₄ und 7,4 % sonstigen Komponenten und mit einer Temperatur von -71,7°C und einem Druck von 0,1 bar abgezogen. Sie kann gegebenenfalls in die Leitung 6 zwischen dem Dehydrierungsreaktor 5 und der Verdichter- und Trocknungseinheit 7 geführt werden. Am Sumpf des Abscheiders S4 fällt mit einer Zusammensetzung von 2,2 % C₃H₆, 4,3 % C₃H₈, 48,8 % i-C₄H₁₀, 39,8 % i-C₄H₈ und 4,9 % sonstigen Komponenten eine flüssige Produktfraktion an, die nach Verdichtung 23 auf einen Druck von 10,9 bar und Erwärmung im Wärmetauscher W1 gegen abzukühlende Verfahrensströme auf eine Temperatur von 40,5°C über Leitung 24 die Anlage verläßt. Zur Erhöhung der Reinheit der am Kopf bzw. Sumpf der Stripperkolonne S3 abgezogenen Fraktionen ist zusätzlich am Kopf der Stripperkolonne S3 ein Rücklauf vorgesehen (in der Figur nicht dargestellt). Hierbei wird unterhalb des Kopfes der Stripperkolonne S3 eine H₂-reiche Gasfraktion abgezogen, im Wärmetausch mit einem Teilstrom des vor dem Wärmetauscher W2 aus Leitung 16 abgezogenen C₄-reichen Stromes teilweise kondensiert und oberhalb der Abzugstelle wieder auf den Kopf der Stripperkolonne S3 gegeben. Dieser zusätzliche Rücklauf dient zur Verbesserung der rektifikatorischen Eigenschaften der Stripperkolonne S3 und ermöglicht ein nahezu vollständiges Ausgasen von Methan aus dem der Stripperkolonne S3 über Leitung 16 zugeführten Gemischstrom.

## Patentansprüche

1. Verfahren zum Auftrennen eines im wesentlichen aus Wasserstoff, Methan und C₃/C₄-Kohlenwasserstoffen bestehenden Einsatzstromes, der zuvor eine Dehydrierung durchgeläufen ist (Vorbehandlung), nachdem der Einsatzstrom eine mindestens einstufige partielle Kondensation und Auftrennung in eine C₃/C₄-Kohlenwasserstoff-reiche Fraktion und in eine H₂/CH₄-Fraktion erfährt, wonach ein Teilstrom der H₂/CH₄-Fraktion dem noch nicht vorbehandelten Einsatzstrom beigemischt wird, **dadurch gekennzeichnet, daß** die aus dem ersten Abscheider (S1) abgezogene H₂/CH₄-Gasfraktion in zwei Teilströme (10, 17) aufgeteilt wird, wobei der erste Teilstrom (10) zusammen mit der Flüssigfraktion (11) des ersten Abscheiders (S1) nach partieller Kondensation (W2) einem zweiten Abscheider (S2) wird zugeführt, die am Sumpf des zweiten Abscheiders (S2) abgezogene C₃/C₄-Kohlenwasserstoff-reiche Flüssigfraktion (16) zusammen mit dem zweiten Teilstrom der H₂/CH₄-Gasfraktion (17) auf dem ersten Abscheider (S1) in eine Stripperkolonne (S3) geleitet wird, daß am Kopf des zweiten Abscheiders (S2) eine H₂-reiche Gasfraktion mit hoher Reinheit (13) abgezogen und nach Verdampfen im Wärmetausch (W2) gegen abzukühlende Verfahrensströme mit dem Einsatzstrom (2, 2a, 2b) vor dessen Vorbehandlung vermischt wird und daß am Sumpf der Stripperkolonne (S3) eine C₃/C₄-reiche Produktfraktion (20) und am Kopf der Stripperkolonne (S3) eine H₂-reiche Gasfraktion mit weniger hoher Reinheit (18) abgezogen werden.

2. Verfahren nach Anspruch 1, dadurch gekennzeichnet, daß die am Kopf des zweiten Abscheiders (S2) abgezogene H₂-reiche Gasfraktion mit hoher Reinheit (13) kälteleistend entspannt wird (T1).

3. Verfahren nach einem der Ansprüche 1 oder 2, dadurch gekennzeichnet, daß das Vermischen der am Kopf des zweiten Abscheiders (S2) abgezogenen H₂-reichen Gasfraktion mit hoher Reinheit (13) mit dem Einsatzstrom (2, 2a, 2b) auf mindestens zwei unterschiedlichen Temperaturniveaus erfolgt.

4. Verfahren nach einem der Ansprüche 1 bis 3, dadurch gekennzeichnet, daß unterhalb des Kopfes der Stripperkolonne (S3) eine H₂-reiche Gasfraktion abgezogen, teilweise kondensiert und oberhalb der Abzugstelle wieder auf den Kopf der Kolonne (S3) gegeben wird.

## Claims

1. Process for the separation of a feed stream essentially comprising hydrogen, methane and C₃/C₄-hydrocarbons which has previously passed through a dehydrogenation (pretreatment), after which the feed stream experiences an at least single-stage partial condensation and separation into a C₃/C₄-hydrocarbon-rich fraction and an H₂/CH₄-fraction, whereupon a part-stream of the H₂/CH₄ fraction is mixed with the still unpretreated feed stream, **characterized in that** the H₂/CH₄ gas fraction taken off from the first separator (S1) is divided into two part-streams (10, 17), the first part-stream (10) together with the liquid fraction (11) of the first separator (S1) being fed after partial condensation (W2) to a second separator (S2), the C₃/C₄-hydrocarbon-rich liquid fraction (16) taken off at the bottom of the second separator (S2) being passed into a stripper column (S3) together with the second part-stream of the H₂/CH₄ gas fraction (17) from the first separator (S1), in that at the top of the second separator an H₂-rich gas fraction having high purity (13) is taken off and after vaporizing in heat exchange (W2) against process streams to be cooled is mixed with the feed stream before pretreatment of the feed stream (2, 2a, 2b) and in that at the bottom of the stripper column (S3) a C₃/C₄-rich product fraction (20) is taken off and at the head of the stripper column (S3) an H₂-rich gas fraction having less high purity (18) is taken off.

2. Process according to Claim 1, characterized in that the H₂-rich gas fraction having high purity (13) taken off at the top of the second separator (S2) is expanded (T1) in a manner producing refrigeration capacity.

3. Process according to one of Claims 1 or 2, characterized in that the mixing of the H₂-rich gas fraction having high purity (13), taken off at the top of the second separator (S2), with the feed stream (2, 2a, 2b) is carried out at at least two different temperature levels.

4. Process according to one of Claims 1 to 3, characterized in that an H₂-rich gas fraction is taken off below the top of the stripper column (S3), partially condensed and reapplied to the top of the column (S3) above the take-off point.

## Revendications

1. Procédé de séparation d'un courant de charge constitué essentiellement d'hydrogène, de méthane et d'hydrocarbures en C₃/C₄, qui est d'abord soumis à une déshydrogénation (traitement préalable), puis le courant de charge subit au moins une condensation partielle et une séparation en une fraction riche en hydrocarbures en C₃/C₄ et en une fraction H₂/CH₄, où un courant partiel de la fraction H₂/CH₄ est mélangé au courant de charge non encore traité préalablement, caractérisé en ce que la fraction gazeuse H₂/CH₄ sortant du premier séparateur (S1) est divisée en deux courants partiels (10, 17), où le premier courant partiel (10), avec la fraction liquide (11) du premier séparateur (S1) est introduit, après condensation partielle (W2), dans un deuxième séparateur (S2), la fraction liquide (16) riche en hydrocarbures en C₃/C₄, sortant du bas du deuxième séparateur (S2), avec le deuxième courant partiel (17) de la fraction gazeuse H₂/CH₄ provenant du premier séparateur (S1), est dirigée vers une colonne de rectification (S3), en ce que, au sommet du deuxième séparateur (S2), une fraction gazeuse riche en H₂ et à pureté élevée (13) sort et, après évaporation dans un échangeur de chaleur (W2) vis-à-vis du courant réfrigérant, est mélangée avec le courant de charge (2, 2a, 2b) avant son traitement préalable, et en ce que l'on prélève au bas de la colonne de rectification (S3), une fraction de produit (20) riche en C₃/C₄, et à la tête de la colonne de rectification (S3) une fraction gazeuse riche en H₂ et à pureté quelque peu plus élevée (18).

2. Procédé suivant la revendication 1, caractérisé en ce que la fraction gazeuse riche en H₂ et à pureté élevée (13), sortant du haut du deuxième séparateur (S2) est détendue à froid (T1).

3. Procédé suivant l'une quelconque des revendications 1 ou 2, caractérisé en ce que le mélange de la fraction gazeuse riche en H₂, et à pureté élevée (13), sortant du deuxième séparateur (S2), avec le courant de charge (2, 2a, 2b), se fait à au moins deux niveaux différents de température.

4. Procédé suivant l'une quelconque des revendications 1 à 3, caractérisé en ce qu'une fraction gazeuse riche en H₂ est prélevée au-dessus de la tête de la colonne de rectification (S3), est partiellement condensée et est à nouveau introduite dans la tête de la colonne (S3), en amont de l'emplacement de détente.
